# EUROPÄISCHE PATENTSCHRIFT

(11) **EP 0 970 664 B1**
(45) Veröffentlichungstag und Bekanntmachung des Hinweises auf die Patenterteilung: **07.06.2006**
(21) Anmeldenummer: 99111555.1
(22) Anmeldetag: 15.06.1999
(51) Int. Cl.: A61F 2/06

(54) **Radial aufweitbare Stützvorrichtung**
Radially expandable stent
Dispositif de support expansible radialement

(30) Priorität: 03.07.1998 DE 19829702
(43) Veröffentlichungstag der Anmeldung: 12.01.2000
(73) Patentinhaber: W.C. Heraeus GmbH, 63450 Hanau (DE)
(72) Erfinder: Herklotz, Günter Dr., 63486 Bruchköbel (DE); Trötzschel, Jens, 63486 Bruchköbel (DE)
(74) Vertreter: Kühn, Hans-Christian

(56) Entgegenhaltungen:
- EP-A- 0 335 341
- EP-A- 0 873 728
- WO-A-99/17680
- US-A- 5 853 419

## Beschreibung

Die Erfindung betrifft eine radial aufweitbare Stützstruktur zur Offenhaltung von Lumina innerhalb eines Körpers, insbesondere eines Blutgefäßes.

EP 0 335 341 B1 betrifft u. a. ein aufweitbares intraluminares vaskuläres Gewebe oder Prothese mit zumindest einem dünnwandigen rohrförmigen Teil mit ersten und zweiten Enden und einer zwischen dem ersten und zweiten Ende angeordneten Wandfläche, die eine im wesentlichen gleichförmige Dicke und mehrere Schlitze aufweist, die im wesentlichen parallel zur Längsachse des rohrförmigen Teils ausgerichtet sind, wobei das rohrförmige Teil einen ersten Durchmesser aufweisen kann, der den intraluminalen Transport des rohrförmigen Teils in einen ein Lumen aufweisenden Körperdurchgang ermöglicht, und wobei das rohrförmige Teil einen zweiten, aufgeweiteten und deformierten Durchmesser aufweisen kann, nach dem vom Inneren des rohrförmigen Teils aus eine radial nach außen gerichtete Kraft aufgebracht ist, wobei der zweite Durchmesser variabel ist und vom Betrag der auf das rohrförmige Teil ausgeübten Kraft abhängt, wodurch das rohrförmige Teil aufgeweitet und deformiert wird, um das Lumen des Körperdurchgangs aufzuweiten. Das vaskuläre Gewebe oder die vaskuläre Prothese weist mehrere rohrförmige Teile und zumindest ein Verbindungsteil auf, welches zwischen aneinander angrenzenden rohrförmigen Teilen angeordnet ist, um aneinander angrenzende rohrförmige Teile biegbar miteinander zu verbinden.

Nachteilig bei diesem intraluminaren vaskulären Gewebe ist die relativ hohe radiale Steifigkeit der einzelnen Teile, was die in einem Lumen notwendige Flexibilität zur Verhinderung von inneren Verletzungen merklich beeinträchtigt.

In US 4,969,458 wird eine intraluminare Stützstruktur offenbart, die aus einer spiralförmigen Feder besteht. Nachteilig hierbei ist die relativ hohe radiale Instabilität, die zu unerwünschten Knickstellen führen kann.

EP873728 hat eine radial aufweitbare Stützvorrichtung zum Gegenstand, bei der mehrere miteinander verbundene Ringe entlang der Längsachse des rohrförmigen Körpers angeordnet sind, dadurch gekennzeichnet, dass die Ringe (2) untereinander mit langgestreckien, im wesentlichen in Längsrichtung des rohrförmigen Körpers verlaufenden Gliedern (4) einer zweiten Gruppe von Gliedern verbunden sind wobei benachbarte Schlitze (5) eines Ringes (2) durch Verbindungsstellen (3) miteinander verbunden sind und die Glieder (4) der zweiten Gruppe von Gliedern durch Verbindungsstellen (3) an ihren Enden jeweils mit einer Verbindungsstelle (3) des benachbarten Ringes (2) verbunden sind. Bei den dortigen Strukturen weisen nicht die Längsaxial- und die Radial-Teilstrukturen jeweils Doppelmäandermuster auf.

Aus dem Vorgenannten ergibt sich das Problem, mit Hilfe einer neuartigen Stützstruktur die oben genannten Nachteile zumindest teilweise zu beseitigen. Das sich ergebende Problem liegt insbesondere darin, eine hohe Stabilität in Längsrichtung bei nicht zu geringer radialer Steifigkeit unter Vermeidung einer Verkürzung der StützstruKtur bei radialer Aufweitung und unter Vermeidung von unkontrollierten radialen Knickbewegungen zu gewährleisten.

Dieses Problem wird erfindungsgemäß durch eine Stützstruktur nach Anspruch 1 gelöst.

Die erfindungsgemäße Stützstruktur weist einen rohrförmigen, mindestens zwei Teilstrukturen aufweisenden Körper mit einer sich zwischen einem ersten und einem zweiten Ende erstrekkenden Wandfläche auf, die mehrere Ausschnitte, insbesondere Schlitze, aufweist, die im wesentlichen parallel zu der Längsachse des rohrförmigen Körpers ausgerichtet sind. Mindestens eine Teilstruktur verläuft ohne Unterbrechung in axialer Richtung zumindest nahezu vom ersten bis zum zweiten Ende des rohrförmigen Körpers. Die erste Teilstruktur ist zumindest in radialer Richtung dehnbar und weist mindestens einen Radial-Dehnungselement-Typ auf, wobei die einzelnen Radial-Dehnungselemente als Ringe oder ringförmig angeordnet sind. Die zweite Teilstruktur ist in axialer Richtung nahezu starr. Der Radial-Dehnungselement-Typ kann geschlossen, beispielsweise oval-, rechteck-, rauten- oder ellipsenförmig, oder offen, beispielsweise mäanderförmig, ausgebildet sein.

Durch insbesondere diese Anordnung der Teilstrukturen wird erreicht, daß bei radialer Aufweitung des rohrförmigen Körpers die zweite Teilstruktur die dabei auftretenden längsaxialen Kräfte aufnimmt. Dies hat zur Folge, daß die radiale von der axialen Deformation unabhängig ist und somit keine Verkürzung in Längsrichtung bei radialer Aufweitung stattfindet. Dies ist bei der invasiven Chirurgie besonders wichtig, um eine gewünschte Stützwirkung über eine bestimmte Länge bereitzustellen.

Mindestens eine Teilstruktur weist mindestens ein Mäandermuster auf, um die Flexibilität zu erhöhen. Dies gilt für die mindestens zwei Teilstrukturen.
Die Schleifen des Mäandermusters sind unterschiedlich groß. Dies bezieht sich auf die Elongation (Amplitude) und/oder "Wellenlänge", sowohl in radialer als auch in Längsrichtung der Stützstruktur. Damit ist insgesamt eine zumindest bezogen auf die erste Teilstruktur hohe homogene Aufweitbarkeit gegeben.
Das Mäandermuster der ersten Teilstruktur ist ein Doppelmäandermuster, um die radiale Aufweitbarkeit zu erhöhen.
Die einzelnen Radial-Dehnungselemente weisen somit ein Doppelmäandermuster auf, das aus einem ersten Mäandermuster mit Schleifen und einem zweiten Mäandermuster mit im Vergleich zu den Schleifen des ersten Mäandermusters größeren oder kleineren Schleifen besteht, um auf diese Art und Weise bei radialer aufweitender Beaufschlagung zumindest ein annähernd gleichmäßiges Aufweiten zu erreichen.

Darüber hinaus ist in vorteilhafter Weise ein jeweils an den Längsenden der ersten Radial-Dehnungselemente angreifender Verbindungs-Typ als schleifenförmiger Steg ausgebildet; der Steg verhindert bzw. vermindert bei Biegebeanspruchung ein Abstehen oder Herausspießen der Schleifen aus der Oberfläche der Stützstruktur. Darüber hinaus verbessern sie im aufgeweiteten Zustand die Abstützwirkung des Stents durch eine gleichmäßigere Oberfläche.

Schließlich ist es vorteilhaft, wenn die Wandfläche eine im wesentlichen gleichförmige Dicke aufweist, um die homogene radiale Ausdehnung zu verbessern.

Die Stützstruktur kann beispielsweise aus einem für medizinische Zwecke geeigneten Edelstahl bestehen und/oder eine biokompatible Beschichtung aufweisen. Darüber hinaus kann jedes biokompatible Material als Werkstoff zur Herstellung von Stents in Frage kommen, z. B. Tantal, Platin, Niob, Legierungen und Kunststoffe. Die Strukturen können durch Laserschneiden, Elektroerosion, Ätzen oder auch Spanabheben hergestellt werden.

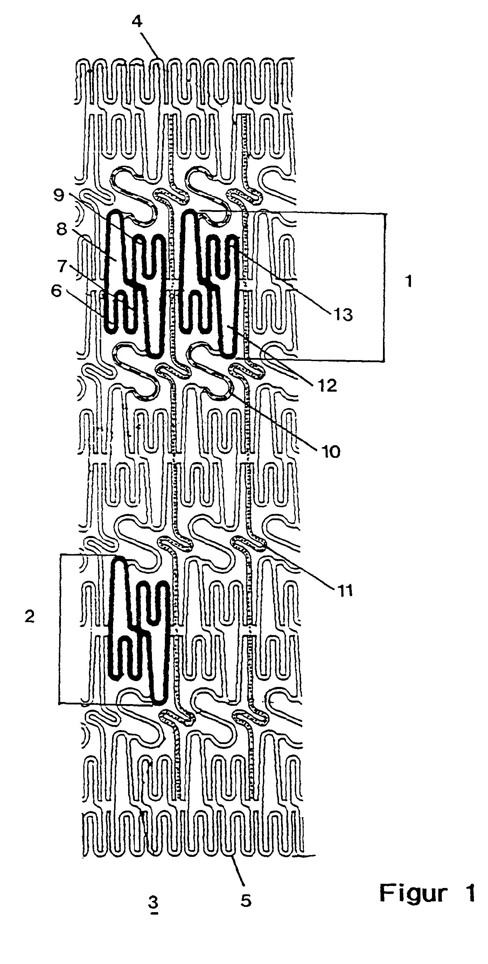

Nachfolgend wird ein Ausführungsbeispiel der Erfindung anhand einer Zeichnung erläutert. In der Zeichnung zeigt
Figur 1 - eine abgerollte Stützstruktur.

Figur 1 zeigt die abgerollte Wandfläche einer Stützstruktur. Diese weist eine erste aus aus Mäanderstrukturen 12 und 13 bestehenden Doppelmäanderstrukturen bestehende Teilstruktur 1, 2 und eine zweite Teilstruktur 11 auf. Die Mäanderstrukturen bilden mehrere Schlitze 6, 7, 8, 9, die im wesentlichen parallel zu der Längsachse des rohrförmigen Körpers 3 ausgerichtet sind. Die zweite Teilstruktur 11 läuft ohne Unterbrechung in axialer Richtung nahezu vom ersten 4 bis zum zweiten Ende 5. Die erste Teilstruktur ist in radialer Richtung dehnbar und weist einen Radial-Dehnungselement-Typ auf, der aus den Radial-Dehnungselementen besteht.
Diese sind als Ringe angeordnet. Die zweite Teilstruktur 11 ist trotz des schleifenförmigen Verlaufs in axialer Richtung relativ starr.

Weiterhin ist der jeweils an den Längsenden der Radial-Dehnungselemente, also in diesem Fall an den Schleifenenden des ersten Mäandermusters 12, angreifender Verbindungstyp 10 als schleifenförmiger Steg ausgebildet.

Der beispielhaft dargestellte Stent weist eine Länge von ca. 15 mm und einen radialen Umfang von ca. 4 mm auf.

Mit dieser beispielhaften Ausgestaltung der erfindungsgemäßen Stützstruktur werden ausgezeichnete Ergebnisse erreicht.

## Patentansprüche

1. Radial aufweitbare Stützstruktur zur Offenhaltung von Lumina innerhalb eines Körpers, insbesondere eines Blutgefäßes, mit einem rohrförmigen, mindestens zwei Teilstrukturen aufweisenden Körper (3) mit einer sich zwischen einem ersten (4) und einem zweiten Ende (5) erstreckenden Wandfläche, die mehrere Ausschnitte (6,7,8,9), insbesondere Schlitze, aufweist, die im wesentlichen parallel zu der Längsachse des rohrförmigen Körpers ausgerichtet sind,
- wobei mindestens eine zweite Teilstruktur (11) ohne Unterbrechung in axialer Richtung zumindest nahezu vom ersten bis zum zweiten Ende des rohrförmigen Körpers verläuft, die erste Teilstruktur (1, 2) zumindest in radialer Richtung dehnbar ist und mindestens einen Radial -Dehnungselement-Typ aufweist, der aus Radial-Dehnungselementen besteht, wobei die einzelnen Radial-Dehnungselemente als Ringe oder ringförmig angeordnet sind und die zweite Teilstruktur (11) in axialer Richtung nahezu starr ist und
- wobei beide Teilstrukturen (1, 2; 11) derart angeordnet sind, dass bei radialer Aufweitung des rohrförmigen Körpers die zweite(n) Teilstruktur(en) (11) die dabei auftretenden längsaxialen Kräfte aufnimmt/nehmen,
die erste Teilstruktur (1, 2) und die zweite(n) Teilstruktur(en) (11) je mindestens ein Mäandermuster (12, 13) aufweisen, wobei
das Mäandermuster (12,13) der ersten Teilstruktur (1, 2) ein Doppelmäandermuster
aus einem ersten Mäandermuster (12) mit Schleifen und einem zweiten Mäandermuster (13) mit im Vergleich zu den Schleifen des ersten Mäandermusters größeren oder kleineren Schleifen besteht.

2. Stützstruktur nach Anspruch 1, **dadurch gekennzeichnet, dass** mindestens ein jeweils an den Längsenden der ersten Radial-Dehnungselemente angreifender Verbindungs-Typ (10) als schleifenförmiger Steg ausgebildet ist.

3. Stützstruktur nach einem der vorhergehenden Ansprüche, **dadurch gekennzeichnet, dass** die Wandfläche eine im wesentlichen gleichförmige Dicke aufweist

## Claims

1. Radially expandable support structure for keeping open lumina within a body, in particular a blood vessel, comprising a tubular body (3) having at least two part structures, with a wall surface which extends between a first (4) and a second end (5) and has a plurality of cut-out areas (6, 7, 8, 9), in particular slots, which are substantially oriented parallel to the longitudinal axis of the tubular body,
- wherein at least a second part structure (11) runs without interruption in the axial direction at least virtually from the first to the second end of the tubular body, the first part structure (1, 2) is expandable at least in the radial direction and has at least one radial-expansion component which comprises radial expansion elements wherein the individual radial expansion elements (12, 13) are arranged as rings or annularly and the second part structure (11) is virtually rigid in the axial direction and
- wherein the two part structure (1, 2; 11) are arranged in such a way that on radial expansion of the tubular body, the second part structure(s) (11) absorbs/absorb the longitudinal axial forces occurring in the process, the first part structure (1, 2) and the second part structure(s) each have at least one meandering pattern (12, 13), the meandering pattern (12, 13) of the first part structure (1, 2) being a double meandering pattern, consisting of a first meandering pattern (12) with loops and second meandering pattern (13) with larger or smaller loops in comparison to the loops of the first meandering pattern.

2. Support structure according to claim 1, **characterised in that** at least one connection type (10) engaging in each case on the longitudinal ends of the first radial expansion elements (12) is configured as a loop-shaped web.

3. Support structure according to one of the preceding claims, **characterised in that** the wall surface has a substantial uniform thickness.

## Revendications

1. Structure de support expansible radialement pour maintenir ouvertes des lumières à l'intérieur d'un corps, notamment d'un vaisseau sanguin, avec un corps (3) tubulaire comportant au moins deux structures partielles, avec une surface de paroi s'étendant entre une première (4) et une seconde extrémité (5) et comportant plusieurs découpes (6, 7, 8, 9), notamment des entailles, qui sont orientées sensiblement parallèlement à l'axe longitudinal du corps tubulaire,
- au moins une deuxième structure partielle (11) s'étendant sans interruption en direction axiale au moins pratiquement de la première à la seconde extrémité du corps tubulaire, la première structure partielle (1, 2) étant extensible au moins en direction radiale et comportant au moins un type d'élément d'extension radiale constitué d'éléments d'extension radiale, les différents éléments d'extension radiale étant disposés en tant qu'anneaux ou sous forme annulaire et la deuxième structure partielle (11) étant pratiquement rigide en direction axiale, et
- les deux structures partielles (1, 2 ; 11) étant disposées de façon à ce qu'en cas d'expansion radiale du corps tubulaire la ou les deuxièmes structures partielles (11) absorbent les forces axiales longitudinales s'exerçant alors,
- la première structure partielle (1, 2) et la ou les deuxièmes structures partielles (11) présentant chacune au moins un motif en méandres (12, 13) et
- le motif en méandres (12, 13) de la première structure partielle (1, 2) étant un double motif en méandres, constitué d'un premier motif en méandres (12) ayant des boucles et d'un deuxième motif en méandres (13) ayant des boucles plus grandes ou plus petites par comparaison aux boucles du premier motif en méandres.

2. Structure de support selon la revendication 1, **caractérisée en ce qu'**au moins un type de liaison (10) s'accrochant respectivement aux extrémités longitudinales des premiers éléments d'extension radiale est conçu sous la forme d'une entretoise en forme de boucle.

3. Structure de support selon l'une des revendications précédentes, **caractérisée en ce que** la surface de paroi présente une épaisseur sensiblement uniforme.
